# EUROPÄISCHE PATENTSCHRIFT

(11) **EP 1 191 927 B1**
(45) Veröffentlichungstag und Bekanntmachung des Hinweises auf die Patenterteilung: **12.03.2003**
(21) Anmeldenummer: 00936897.8
(22) Anmeldetag: 20.06.2000
(51) Int. Cl.: A61K 9/70

(54) **MIKRORESERVOIRSYSTEM AUF BASIS VON POLYSILOXANEN UND AMBIPHILEN LÖSEMITTELN**
MICRORESERVOIR SYSTEM ON THE BASIS OF POLYSILOXANES AND AMBIPHILIC SOLVENTS
SYSTEME A MICRORESERVOIR, A BASE DE POLYSILOXANES ET DE SOLVANTS HYDROPHILES ET LIPOPHILES

(30) Priorität: 02.07.1999 DE 19930340; 04.12.1999 DE 19958554
(43) Veröffentlichungstag der Anmeldung: 03.04.2002
(73) Patentinhaber: LTS Lohmann Therapie-Systeme AG, 56626 Andernach (DE)
(72) Erfinder: MÜLLER, Walter, D-56564 Neuwied (DE)
(74) Vertreter: Schmidt, Werner, Dr.
(86) Internationale Anmeldenummer: EP0005658
(87) Internationale Veröffentlichungsnummer: WO01001967

(56) Entgegenhaltungen:
- WO-A-87/07138
- WO-A-90/10425
- WO-A-94/06383

## Beschreibung

Transdermale therapeutische Systeme (TTS) können unter Vernachlässigung von wenig gebräuchlichen Sonderformen in zwei Grundtypen unterschieden werden, die sogenannten Matrixsysteme und die sogenannten Reservoirsysteme.

Bei den sogenannten Matrixsystemen ist im einfachsten Fall der Wirkstoff in einer selbstklebenden Schicht gelöst bzw. zum Teil auch nur suspendiert oder dispergiert in Form von Kristallen.

Die von den Matrixsystemen zu unterscheidenden Reservoirsysteme stellen eine Art Beutel aus einer inerten Rückschicht und einer wirkstoffdurchlässigen Membran dar, wobei sich der Wirkstoff in einer flüssigen Zubereitung in diesem Beutel befindet. Meistens ist die Membran mit einer Kleberschicht versehen, die der Verankerung des Systems auf der Haut dient.

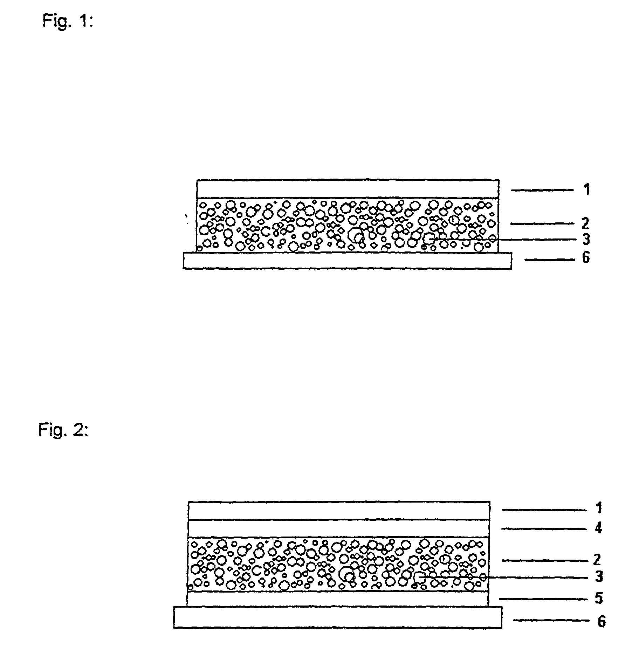

Systeme mit flüssigen Mikroreservoiren können gewissermaßen als Zwitter aus den beiden Grundformen angesehen werden. Auch hier befindet sich der Wirkstoff zum größten Teil nicht in den polymeren Bestandteilen des Systems, sondern in den flüssigen Mikroreservoiren, die in die Polymerschichten eingebettet sind. In ihrem einfachsten Fall sind die flüssigen Mikroreservoire in eine selbstklebenden Polymerschicht eingebettet, wobei der Kleber dann selbst als eine Art Membran aufgefaßt werden kann. Ein so gestaltetes System ist rein äußerlich nicht von einem gewöhnlichen Matrixsystem zu unterscheiden. Erst bei der mikroskopischen Betrachtung sind die Mikroreservoire und damit die heterogene Struktur des Kleberfilms zu erkennen. Ein solches System in seiner einfachsten Ausführung ist in Figur 1 dargestellt.

Ist jedoch die so mit Wirkstoff beladene Schicht nicht oder nicht ausreichend selbstkiebend, kann eine weitere geeignete selbstklebende Schicht, die zur Verankerung des Systems auf der Haut dient, aufgebracht werden. Die gleiche Maßnahme kann dann nötig sein, um die Rückschicht des Systems besser auf der wirkstoffbeladenen Schicht zu verankern. Ein solches System mit zwei zusätzlichen Kleberschichten ist in Figur 2 dargestellt. Natürlich ist auch bei solchen Systemen die Möglichkeit gegeben, hautseitig die wirkstoffbeladene Schicht mit einer Steuermembran und dann gegebenenfalls diese Membran hautseitig mit einer Hautkleberschicht zu versehen. Auch diese Hautkleberschicht kann dann zur Abgabe einer Initialdosis mit Mikroreservoiren ausgestattet sein.

Bevorzugtes Polymer für Mikroreservoirsysteme sind Polysiloxane. Polysiloxane haben nur ein geringes Lösevermögen für Wirkstoffe. Dies bedeutet, daß die Wirkstoffe in Polysiloxanen ohne Zusätze zum größten Teil nur dispergiert und nicht im Polymer gelöst vorliegen.

Durch die Verwendung von Mikroreservoiren mit physiologisch akzeptablen Lösemitteln für den einzuarbeitenden Wirkstoff kann die Beladung mit gelöstem Wirkstoff wesentlich verbessert werden.

Wirkstoffabgabesysteme mit Mikroreservoiren sind beschrieben in den US-Patentschriften 3,946,106 und 4,053,580, bei denen als Basis für die sehr hydrophilen Flüssigreservoire Polyethylenglykol, Propylenglykol oder 1,3-Butandiol in Abmischung mit Wasser und als Polymer ein spezielles in-situ vernetzbares Zweikomponentenpolysiloxan eingesetzt wird. Die in diesen beiden Patentschriften beschriebenen Systeme sind jedoch für die transdermale Applikation nicht vorgesehen und ungeeignet.

In der US-Patentschrift 4,814,184 ist ein transdermales System auf Basis von einem Polysiloxan, einem Emulgator auf Basis einer polyoxethylierten Organopolysiloxanverbindung und einem polaren hydrophilen Wirkstoff, gelöst in einer hydrophilen Flüssigkeit, beschrieben. Speziell genannt als Lösemittel für den hydrophilen polaren Wirkstoff werden Polyethylenglykole mit einem Molekulargewicht zwischen 200 und 2000. Der Nachteil dieses Systems ist es, daß ein Emulgator benötigt wird und polare hydrophile Lösemittel nur hydrophile polare Wirkstoffe in genügender Menge lösen. Sie sind damit nicht geeignet für Wirkstoffe mit mittlerer Polarität, die gerade wegen dieser Eigenschaft für die transdermale Verabreichung besonders gut geeignet sind.

In der US-Patentschrift 5,145,682 ist ein System für Estradiol und Estradiolderivate, gegebenenfalls in Kombination mit einem Gestagen beschrieben, bei dem wasserunlösliche bzw. mit Wasser nicht mischbare permeationsfördernde Mittel - speziell genannt ist n-Dodecylalkohol - in Form von Mikroreservoiren in eine selbstklebende Polymerschicht eingearbeitet sind. Auch solche sehr lipophilen Substanzen wie mittel- und langkettige Alkohole sind keine guten Lösemittel für Wirkstoffe mit mittlerer Polarität und damit auch nicht für das in dieser Patentschrift ausdrücklich erwähnte Estradiol. Ihre Aufgabe ist es daher nicht, den Wirkstoff zu lösen, sondern lediglich als permationsfördemde Mittel zu wirken und die Barrierefunktion des Stratum Corneums zu reduzieren.

Aufgabe der vorliegenden Erfindung ist es nun, durch die Verwendung von geeigneten physiologisch akzeptablen Lösemitteln die Beladung von Silikonklebern mit gelösten Wirkstoffen mittlerer Polarität zu verbessern und damit den Einsatzbereich von Silikonklebem und Mikroreservoirsystemen zu erweitern.

Dies gelingt erfindungsgemäß dadurch, daß zur Bildung von Mikroreservoiren ambiphile, bevorzugt bei Raumtemperatur flüssige dipolare organische Lösemittel verwendet werden, die aufgrund ihrer physikalisch-chemischen Eigenschaften nur eine beschränkte Mischbarkeit mit Silikonpolymeren aufweisen und zusätzlich zu einem gewissen Grad, vorzugsweise mindestens im Gew.-Verhältnis von einem Teil Lösemittel mit 3 Teilen Wasser, z. B. 1:1 mit Wasser mischbar sind.

Der Begriff "ambiphile Lösemittel" besagt, was durch den Wortteil "ambi" ausgesagt wird, daß diese Stoffe eine zweifache Philie aufweisen, nämlich sowohl eine gewisse Hydrophilie als auch eine gewisse Lipophilie. Es handelt sich bei ihnen in erster Linie um dipolare organische Lösemittel. Die Mischbarkeit mit Silikonpolymeren beträgt zweckmäßig nicht mehr als 20 Gew.-%

Ambiphile Lösemittel stehen bezüglich ihrer Eigenschaften zwischen den sehr polaren Lösemitteln wie Wasser und den sehr lipophilen Lösemitteln wie Alkanen, niederen Fettalkoholen (mit 6-12 C-Atomen) und Dieethylether. Das heißt, daß sie zu einem gewissen Grad mit organischen Flüssigkeiten wie Ethylacetat und hydrophilen Lösemitteln wie Methanol oder Wasser mischbar sind und damit ein gutes Lösevermögen für nicht zu lipophile und nicht zu hydrophile Substanzen, also Wirkstoffe mittlerer Polarität haben.

Die Mikroreservoirsysteme, die unter Verwendung solcher ambiphilen, insbesondere dipolaren organischen Lösemittel im Sinne dieser Erfindung hergestellt sind, können generell wie folgt charakterisiert werden:

Transdermales therapeutisches System, umfassend eine für Wirkstoffe undurchlässige Rückschicht, zumindest eine Polymerschicht mit darin enthaltenen, d. h. dispergierten Mikroreservoiren und mindestens einem Wirkstoff und eine vor Gebrauch zu entfernende Schutzschicht, dadurch gekennzeichnet, daß
- der Polymeranteil der Polymerschicht zumindest zu 70, vorzugsweise zumindest zu 80 Gew.-%, aus Polysiloxanen besteht,
- die Mikroreservoire den Wirkstoff in gelöster Form enthalten,
- das Lösemittel für den Wirkstoff mindestens 50, vorzugsweise mindestens 80 Gew.-% eines ambiphilen Lösemittels enthält,
- das ambiphile Lösemittel zu nicht mehr als etwa 20 Gew.-% in Polysiloxanen löslich ist.

Vorzugsweise ist das ambiphile Lösemittel mit Wasser zumindest in einem Gewichtsverhältnis von einem Teil Lösemittel zu 3 Teilen Wasser mischbar.

Die beschränkte Mischbarkeit mit Polysiloxanen beruht auf den polaren Eigenschaften der ambiphilen, insbesondere dipolaren Lösemittel und ist ein wichtiges Kriterium, da es einerseits die Ausbildung von Mikroreservoiren erst erlaubt und andererseits vermeidet, daß wegen zu hoher Mischbarkeit die Kohäsion der aus Polysiloxanen gebildeten Filme in nicht zu akzeptierender Weise geschädigt wird. Eine Wassermischbarkeit von mindestens etwa 25 Gew.-%, z. B. 1:1, ist ebenfalls Ausdruck des Charakters dieser Lösemittel. Sie sind dadurch in der Lage, Wirkstoffe mit mittlerer Polarität, die die Mehrzahl der Wirkstoffe mit Eignung zur transdermalen Anwendung'repräsentieren, in der notwendigen Konzentration zu lösen.

Geeignete Lösemittel für den Wirkstoff können unter Verbindungen gefunden werden, die dadurch charakterisiert sind, daß sie über mindestens eine freie Hydroxylgruppe und mindestens einen weiteren Ethersauerstoff oder zumindest über 2 freie Hydroxylgruppen verfügen.

Die begrenzte Löslichkeit in Polysiloxanen (höchstens 20 Gew.-% ) kann experimentell wie folgt bestimmt werden: Zu einer Lösung des Polysiloxans werden, bezogen auf den Feststoff, etwa 20 Gew.-% des zu testenden Lösemittels gegeben; die Mischung wird schnell gerührt und anschließend auf eine transparente Folie beschichtet. Das Lösemittel des Polysiloxans wird nun bei einer 40°C nicht überschreitenden Temperatur entfernt. Der resultierende Film wird anschließend unter dem Mikroskop auf Tröpfchen des zu testenden Lösemittels untersucht. Sind Tröpfchen zu erkennen, ist damit gesichert, daß die Löslichkeit unterhalb von 20 Gew.-% liegt.

Beispiele für solche Lösemittel sind die verschiedenen Butandiole, insbesondere das 1,3-Butandiol, Dipropylenglykol, Tetrahydrofurfurylalkohol, Diethylenglykoldimethylether, Diethylenglykolmonoethylether, Diethylenglykolmonobutylether, Propylenglykol, Dipropylenglykol, Carbonsäureester von Tri- und Diethylenglykol, polyoxyethylierte Fettalkohole von 6-18 C-Atomen.

Zur Erzielung der für den jeweiligen Wirkstoff idealen Sättigungslöslichkeit können diese Lösemittel auch in Abmischungen verwendet werden. Idealerweise sind die Mikroreservoire bis auf die in ihnen enthaltenen Wasserspuren und den während der Herstellung nicht vermeidbaren Wassereintrag frei von Wasser. Trotzdem kann es in Einzelfällen von Vorteil sein, dem Lösemittel Wasser zur Erniedrigung oder Erhöhung der Löslichkeit der Wirkstoffe in gewissen Mengen beizumischen.

Im allgemeinen haben diese Lösemittel einen Siedepunkt von über 80 °C, insbesondere über 110 °C unter Normalbedingungen. Dies ist keine strikte Einschränkung, macht es aber einfacher, das Lösemittel des Polysiloxans während des Herstellprozesses relativ selektiv zu entfernen, ohne das Lösemittel der Mikroreservoire in nicht mehr akzeptablen Mengen mit abzuziehen.

Den ambiphilen Lösemitteln können kleinere Anteile an Zusätzen wie Tri- und Partialglyceride mittlerer und höherer Fettalkohole und Fettsäuren (C₁₂ - C₂₂) sowie die weiter unten genannten Hilfsstoffe (außer Füllstoffen) zugemischt werden.

Zur Herstellung der Systeme wird der Wirkstoff in dem für ihn geeigneten Lösemittel, bzw. Lösemittelgemisch, gelöst und diese Lösung zu der Lösung des Polysiloxans gegeben. Neben dem ambiphilen und in dem System verbleibenden Lösemittel können dabei auch zusätzlich niedrig siedende Lösemittel wie Ethanol verwendet werden, die später zusammen mit den Lösemitteln des Polysiloxans entfernt werden. Durch schnelles Rühren wird nun die Lösung des Wirkstoffs in der Lösung des Polymers dispergiert. Die resultierende Dispersion wird auf eine abhäsiv (dehesiv) ausgerüstete Folie, z.B. mit einem Erichsen-Rakel, in der gewünschten Dicke beschichtet und das Lösemittel des Polymers bei Temperaturen von 25 - 100 °C, bevorzugt zwischen 30 und 80 °C entfernt. Naturgemäß sollte in jedem Falle der Siedepunkt des ambiphilen Lösemittels über dem des Lösemittels für das Polysiloxan liegen, zweckmäßig mindestens 10, vorzugsweise mindestens 30 °C. Anschließend wird der getrocknete Film mit einer als Rückschicht dienenden Folie kaschiert. Dann werden die Systeme ausgestanzt. Ist der resultierende Film nicht oder nur ungenügend klebend, kann er nach Standardverfahren mit einer zusätzlichen Hautkleberschicht und einer Verankerungsschicht zur Rückschicht ausgestattet werden.

Selbstverständlich können, wenn vorteilhaft, in das System weitere Hilfsstoffe wie permeationsfördemde Stoffe, Füllstoffe, viskositätsbeeinflussende Verbindungen, Kristallisationsinhibitoren oder pH-regulierende Substanzen eingearbeitet werden.

Permeationsfördernde Stoffe dienen dazu, die Barriereeigenschaften des Stratum Corneums im Sinne einer Erhöhung der Wirkstoffdurchlässigkeit zu beeinflussen. Solche Substanzen sind dem Fachmann wohlbekannt und es muß - wenn notwendig - durch Permeationsstudien der für den jeweiligen Wirkstoff geeignete Stoff gefunden werden.

Füllstoffe wie Silicagele, Titandioxid und Zinkoxid können in Verbindung mit dem Polymer eingesetzt werden, um einige physikalische Parameter wie Kohäsion und Klebkraft in der gewünschen Art und Weise beeinflussen.

Viskositätserhöhende Substanzen werden bevorzugt in Verbindung mit der Wirkstofflösung eingesetzt. So wurde gefunden, daß die Dispersion der Wirkstofflösung in der Lösung des Polymers durch eine etwas erhöhte Viskosität der Wirkstofflösung erleichtert wird und zusätzlich die Dispersion an Stabilität gewinnt. Geeignete Substanzen zur Erhöhung der Viskosität der Wirkstofflösung sind z.B. Cellulosederivate wie Ethylcellulose, Hydroxypropylcellulose und hochmolekulare Polyacrylsäuren bzw. deren Salze und/oder Derivate wie Ester.

Die bevorzugte Größe der Mikroreservoire reicht von 5 - 50 µm und hängt im wesentlichen von der Dicke der die Mikroreservoire enthaltenden Schicht ab. Generell kann gesagt werden, daß die maximale Größe der Mikroreservoire 80 % der Dicke der Polymerschicht nicht überschreiten soll. Besonders bevorzugt wird eine Größe zwischen 5 und 30, insbesondere 10 und 25 µm, da diese Größe verträglich ist mit den üblichen Dicken wirkstoffbeladener Filme.

pH-Regulierende Substanzen werden vielfach in Verbindung mit der Wirkstofflösung eingesetzt, da Wirkstoffe mit sauren oder basischen Gruppen eine stark pHabhängige Löslichkeit und Permeationsrate durch die menschliche Haut haben. Durch den pH-Wert kann deshalb die Abgaberate unter in-vivo-Bedingungen gesteuert werden.

Da die ambiphilen Lösemittel im Sinne dieser Erfindung fast alle einen bei Raumtemperatur nicht ganz zu vernachlässigenden Dampfdruck besitzen, ist es wichtig, daß die Systeme während der Lagerung kein Lösemittel verlieren. Es ist deshalb wichtig, daß das Primärpackmittel sehr dicht gegenüber dem Lösemittel für den Wirkstoff ist und die inneren Schichten des Packstoffmaterials nur sehr begrenzt dieses Lösemittel aufnehmen. Als Primärpackmittel für transdermale therapeutische Systeme werden in den meisten Fällen heißsiegelbare Folienverbunde benutzt. Als besonders geeignet für diese speziellen Systeme sind Folienverbunde, die eine geschlossene Aluminiumfolie besitzen und deren innere heißsiegelbare Schicht sehr dünn ist bzw. aus Barex besteht.

Barex-Harze sind gemäß M.Th. Schuler "Kunststoffe-Plastics" 9/1974, Seiten 13-20 thermoplastisch verarbeitbare Barriere-Kunststoffe auf Acrylnitril-Basis, die durch Copolymerisation von Acrylnitril mit ausgesuchten Monomeren hergestellt sind und sich durch besondere chemische Beständigkeit auszeichnen. Diese Kunststoffe zeigen sehr gute Sperreigenschaften gegen verschiedene Gase wie Sauerstoff, Kohlendioxid, Stickstoff sowie viele chemische Agenzien wie Säuren, Alkalien und Lösemittel. Speziell ist Barex ein mit einem Butadien-Acrylnitril-Elastromer modifiziertes Acrylnitril-Methylacrylat-Copolymer. Wichtige Barex-Produkte sind durch eine Propfcopolymerisation von 73-77 Gewichtsteilen Acrylnitril und 23-27 Gewichtsteilen Methylacrylat in Gegenwart von 8-10 Gewichtsteilen Butadien-Acrylnitril-Copolymer mit einem Gehalt von etwa 70 Gew.-% Butadien hergestellt.

Geeignete Silikonpolymere werden von verschiedenen Herstellern geliefert. Als besonders geeignet haben sich Polydimethylsiloxane der Fa. Dow Coming erwiesen, die auch in einer aminresistenten Variante geliefert werden. Die aminresistente Variante verfügt über keine freien Silanolgruppen, die in Gegenwart von basischen Wirkstoffen weitere Kondensationsreaktionen eingehen können.

Die Polysiloxane werden als Lösung in unterschiedlichen Lösemitteln geliefert. Als besonders geeignet haben sich Lösungen in niedrigsiedenden Alkanen, insbesondere n-Hexan und n-Heptan gezeigt. Der besondere Vorteil dieser Lösemittel ist, daß sie als sehr lipophile unpolare Lösemittel nur sehr begrenzt mit den die Mikroreservoire bildenden ambiphilen, insbesondere dipolaren Lösemitteln mischbar sihd und einen genügend hohen Dampfdruck besitzen, der es erlaubt, sie bei moderaten Temperaturen zu entfernen, so daß das ambiphile Lösemittel für die Wirkstoffe in genügender Menge im System verbleibt. Durch die begrenzte Mischbarkeit der ambiphilen Lösemittel mit n-Hexan und n-Heptan kommt es bei Entfernung dieser Lösemittel zu keinen Phasentrennungen, und die in der noch nicht getrockneten, für die Beschichtung vorgesehenen Masse vorgefundene Größenverteilung der wirkstoffbeladenen Tröpfchen des ambiphilen Lösemittels wird in etwa gleicher Größe auch im getrockneten Film vorgefunden.

Polysiloxane haben eine gewisse Neigung zum sogenannten kalten Fluß. Damit ist gemeint, daß sich solche Polymere wie sehr viskose Flüssigkeiten verhalten können und aus dem Rand der Systeme austreten. Dieser kalte Fluß kann erfolgreich durch Füllstoffe wie z.B. Silicagel vermindert werden.

Polysiloxane können selbstklebend sein. Sie sind nur beschränkt mit klebrigmachenden Zusätzen mischbar. Trotzdem kann es im Einzelfall von Vorteil sein, die Klebrigkeit durch den Zusatz geringer Mengen von Klebrigmachem (tackifier) wie Polyterpenen, Kolophoniumderivaten oder Silikonölen zu verbessern.

Als Material für die Rückschicht kommen Folien in Frage, die z.B. aus Polyethylen, Polypropylen, Polyestem wie Polyethylenterephthalat, einem Copolymer aus Ethylen und Vinylacetat (EVA) und Polyvinylchlorid bestehen. Solche Folien können auch aus Laminaten unterschiedlicher Polymerer bestehen und zusätzlich Farbschichten und/oder Farbpigmente enthalten. Solche Folien sind dem Fachmann wohlbekannt, und es kann ohne Problem die für den jeweiligen Zweck beste Folie gefunden werden.

Als Material für die wiederentfernbare Schutzfolie kommen vor allem für Silikonkleber abhäsiv ausgerüstete Polyethylenterephthalatfolien in Frage.

Systeme im Sinne dieser Erfindung zeichnen sich durch eine gute Wirkstoffabgabe bei Applikation auf der Haut aus. Dies ist darauf zurückzuführen, daß die ambiphilen Lösemittel während des Tragens Wasser aus der Haut aufnehmen und sich dieses Wasser aufgrund der sehr lipophilen Natur der Polysiloxane in den Mikroreservoiren sammelt. Durch diese Wasseraufnahme reduziert sich die Sättigungslöslichkeit des Wirkstoffs in den Mikroreservoiren, was zu einer erhöhten bzw. trotz Wirkstoffabgabe relativ konstanten thermodynamischen Aktivität des Wirkstoffs führt.

Ein weiterer Faktor, der zu einer hohen bzw. konstanten thermodynamischen Aktivität des Wirkstoffs während der Applikationszeit führt, ist die Tatsache, daß ambiphile Lösemittel im Sinne dieser Erfindung selbst transdermal resorbiert werden. Dadurch wird die Menge des sich während der Tragezeit noch im System befindlichen Lösemettels geringer und damit die thermodynamische Aktivität des Wirkstoffs entsprechend erhöht bzw. trotz Wirkstoffabgabe auf einem hohen Niveau gehalten.

Bezüglich der Art des Wirkstoffs gibt es eigentlich nur die Einschränkung, daß er sich, bezogen auf die sich aus der Dosis und die beabsichtigte Verwendungsdauer notwendige Menge, in die mit Mikroreservoiren ausgestattete Polysiloxanschicht des transdermalen therapeutischen Systems einarbeiten läßt. Als Obergrenze ergibt sich aus prakischen Erwägungen heraus eine Tageshöchstdosis von etwa 10 mg.

Beispielhaft seien genannt: Hormone wie Estradiol und seine Derivate, Gestagene wie Norethisteronacetat und Levonorgestrel, Androgene wie Testosteron und seine Derivate, β-Blocker wie Bupranolol und Carvedilol, Calciumantagonisten wie Nimodipin, Nifedipin und Lacidipin, ACE-Hemmer wie Captopril, Antiemetika wie Scopolamin, Psychopharmaka wie Haloperidol, Fluoxetin, Mianserin, Amitriptylin, Clomipramin und Paroxetin, Schmerzmittel wie Buprenorphin und Fentanyl, Antiasthmatika wie Salbutamol und Tolubuterol, Antiparkinsonmittel wie Biperiden und Selegilin, Muskelrelaxantia wie Tizanidin, Antihistaminika wie Dimethinden, Doxylamin, Alimemazin und Carbinoxamin.

Zusammenfassend läßt sich sagen, daß Systeme im Sinne dieser Erfindung vorteilhaft geeignet sind für die transdermale Verabreichung von Wirkstoffen mit mittlerer Polarität und einer ca. 10 mg nicht übersteigenden Tagesdosis.

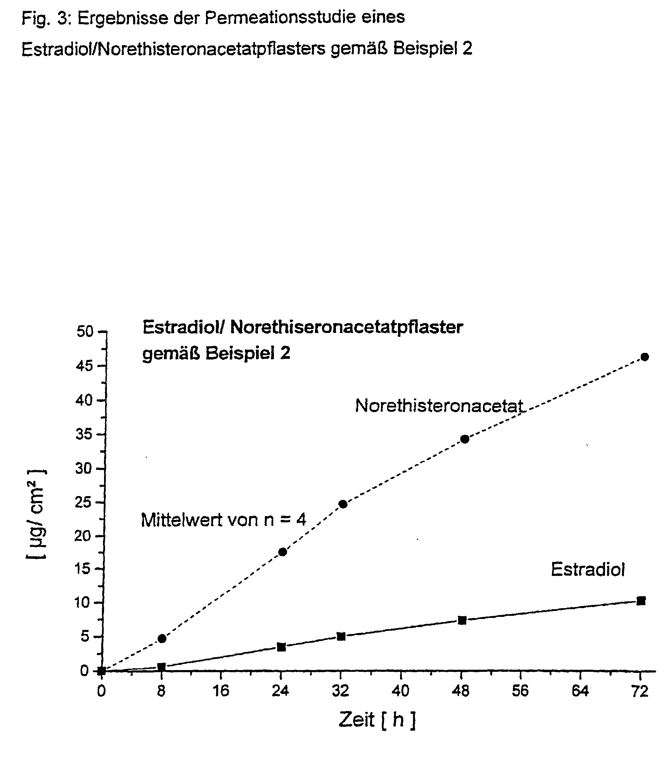

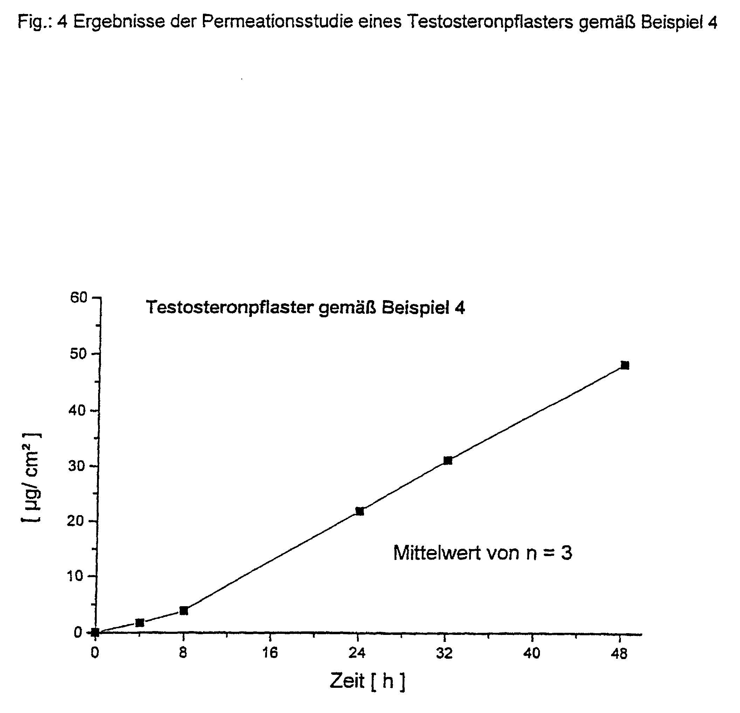

in den folgenden Beispielen wird die Herstellung einiger typischer Systeme beschrieben. Mit einigen Systemen, hergestellt wie in den Beispielen 2 und 4 beschrieben, wurden in-vitro Permeationsstudien unter Verwendung von dem Fachmann bekannten Franz-Diffusionszellen und menschlicher Epidermis durchgeführt. Die Ergebnisse dieser Studien sind in den Fig. 3 und 4 graphisch dargestellt.

### Beispiel 1:

1,0 g Estradiol-hemihydrat werden in 10,0 g Diethylenglykolmonoethylether gelöst. Diese Lösung wird durch schnelles Rühren in 55,0 g eines aminresistenten Polydimethylsiloxans (BIO-PSA 4201 der Fa. Dow Corning; 73% Feststoffgehalt) dispergiert. Diese Masse wird mit einem Erichsen-Rakel auf eine abhäsiv ausgerüstete Polyethylenterephtalatfolie (Scotchpak 1022 Fa. 3M) in einer Dicke von 400 um beschichtet und das Lösemittel durch 20-minütiges Trocknen bei ca. 45°C entfernt.
Der getrocknete Film wird mit der Rückschicht laminiert (Scotchpak 1220 der Fa. 3M). Daraus werden die Pflaster gestanzt und in Beuteln des Primärpackstoffs eingesiegelt.

### Beispiel 2:

0,05 g Estradiol-hemihydrat und 0,5 g Norethisteronacetat werden in 4,5 g Diethylenglykolmonoethylether gelöst. Diese Lösung wird durch schnelles Rühren in 20,5 g eines aminresistenten Polydimethylsiloxans (BIO-PSA 4301der Fa. Dow Corning, 73% Feststoffgehalt) dispergiert. Diese Masse wird mit einem Erichsen-Rakel in einer Dicke von 400 um auf eine abhäsiv ausgerüstete Folie (Scotchpak 1022) beschichtet und das Lösemittel durch 20-minütiges Trocknen bei ca. 45°C entfernt. Der getrocknete Film wird anschließend mit der Rückschicht (Scotchpak 1220) laminiert.

BIO-PSA 4301 wird in einer Dicke von 50 µm auf eine abhäsiv ausgerüstete Folie (Scotchpak 1022) beschichtet und das Lösemittel durch 20-minütiges Trocknen bei ca. 45°C entfernt. Nun wird von dem zuerst hergestellten wirkstoffbeladenen Film die Schutzfolie (Scotchpak 1022) entfernt und der Film auf die im zweiten Schritt hergestellte Haftkleberschicht für die Haut laminiert. Aus dem resultierenden Gesamtlaminat werden nun die Pflaster gestanzt und in Beuteln des Primärpackstoffs eingesiegelt.

### Beispiel 3:

1,0 g Bupranolol werden in 3,0 g Tetrahydrofurfurylalkohol gelöst. Diese Lösung wird durch schnelles Rühren in 21,9 g einer BIO-PSA 4301-Lösung (73% Feststoffgehalt) dispergiert. Diese Masse wird mit einem Erichsen-Rakel auf eine abhäsiv ausgerüstete Folie (Scotchpak 1022) in einer Dicke von 400 µm beschichtet und das Lösemittel durch 20-minütiges Trocknen bei ca. 45°C entfernt. Der getrocknete Film wird mit der Rückschicht laminiert (Scotchpak 1220). Daraus werden die Pflaster gestanzt und in Beuteln des Primärpackstoffs eingesiegelt.

### Beispiel 4:

1,0 g Testosteron, 1,0 g Nicotinsäureamid und 0,4 g Ölsäure werden in 6,2 g Diethylenglykolmonoethylether und 6,2 g 1,3-Butandiol gelöst. Diese Lösung wird durch schnelles Rühren in 60 g einer BIO-PSA 4201-Lösung (73% Feststoffgehalt) dispergiert. Diese Masse wird mit einem Erichsen-Rakel in einer Dicke von 400 µm auf eine abhäsiv ausgerüstete Folie (Scotchpak 1022) beschichtet und das Lösemittel durch 20-minütiges Trocknen bei ca. 45°C entfernt. Der getrocknete Film wird anschließend mit der Rückschicht (Scotchpak 1220) laminiert .

BIO-PSA 4301 wird in einer Dicke von 50 µm auf eine abhäsiv ausgerüstete Folie (Scotchpak 1022) beschichtet und das Lösemittel durch 20-minütiges Trocknen bei ca. 45°C entfernt. Nun wird von dem zuerst hergestellten wirkstoffbeladenen Film die Schutzfolie (Scotchpak 1022) entfernt und der Film auf die im zweiten Schritt hergestellte Haftkleberschicht laminiert. Aus dem resultierenden Gesamtlaminat werden nun die Pflaster gestanzt und in Beuteln des Primärpackstoffs eingesiegelt.

In den Figuren 1 bis 4 haben die Ziffern folgende Bedeutung:
(1) = Rückschicht
(2) = Polymerschicht
(3) = wirkstoffhaltige Mikroreservoire
(4) = Verankerungsschicht
(5) = Hautkleberschicht
(6) = Schutzschicht

## Patentansprüche

1. Transdermales therapeutisches System, umfassend eine für Wirkstoffe undurchlässige Rückschicht, mindestens eine Polymerschicht mit darin enthaltenen Mikroreservoiren und mindestens einem darin gelösten Wirkstoff, sowie eine vor Gebrauch zu entfernende Schutzschicht, **gekennzeichnet durch** die Kombination folgender Merkmale:
a) der Polymeranteil der Polymerschicht besteht mindestens zu 70, vorzugsweise mindestens zu 80 Gew.-% aus löslichen Polysiloxanen,
b) das Lösemittel für den Wirkstoff besteht zu mindestens 50, vorzugsweise zu mindestens 80 Gew.-% aus einem ambiphilen, insbesondere dipolaren, organischen Lösemittel und
c) das ambiphile Lösemittel ist zu nicht mehr als etwa 20 Gew.-% in Polysiloxan löslich und mit Wasser zumindest bis zu einem Gewichtsverhältnis von 1:3 mischbar.

2. Transdermales therapeutisches System gemäß Anspruch 1, **dadurch gekennzeichnet, daß** das Polysiloxan aminresistent ist.

3. Transdermales therapeutisches System gemäß Anspruch 1 oder 2, **dadurch gekennzeichnet, daß** die Mikroreservoire nach Herstellung im wesentlichen frei von Wasser sind.

4. Transdermales therapeutisches System gemäß einem oder mehreren der Ansprüche 1 bis 3, **dadurch gekennzeichnet, daß** das Polysiloxan selbstklebend ist und gegebenenfalls wenigstens einen Füllstoff enthält.

5. Transdermales therapeutisches System gemäß einem oder mehreren der Ansprüche 1 bis 4, **dadurch gekennzeichnet, daß** die mikroreservoirhaltige Schicht zumindest mit einer weiteren, mikroreservoirfreien, selbstklebenden Schicht zur Verankerung auf der Haut und/oder zur Verankerung mit der Rückschicht versehen ist.

6. Transdermales therapeutisches System gemäß einem oder mehreren der Ansprüche 1 bis 5, **dadurch gekennzeichnet, daß** das ambiphile Lösemittel bei Raumtemperatur flüssig ist, zweckmäßig einen Siedepunkt unter Normalbedingungen von über 80 °C, insbesondere über 110 °C, aufweist. vorzugsweise Diethylenglykolmonoethylether, Diethylenglykoldimethylether, eines der Butandiole, Tetrahydrofurfurylalkohol, Dipropylenglykol, Propylenglykol oder eine Mischung davon ist und zweckmäßig zu nicht mehr als 20 Gew.-% in n-Hexan oder n-Heptan löslich ist.

7. Transdermales therapeutisches System gemäß einem oder mehreren der Ansprüche 1 bis 6, **dadurch gekennzeichnet, daß** der Siedepunkt des ambiphilen Lösemittels über dem des Lösemittels für das Polysiloxan liegt, zweckmäßig mindestens 10, vorzugsweise mindestens 30 °C.

8. Transdermales therapeutisches System gemäß einem oder mehreren der Ansprüche 1- 7, **dadurch gekennzeichnet, daß** die maximale Größe der Mikroreservoire 80 % der Dicke der Polymerschicht nicht überschreitet, wobei die Mikroreservoire einen Durchmesser von durchschnittlich 5 - 50, bevorzugt von 5 - 25 µm aufweisen.

9. Transdermales therapeutisches System gemäß einem oder mehreren der Ansprüche 1 bis 8, **dadurch gekennzeichnet, daß** die Mikroreservoire neben dem Wirkstoff und dem ambiphilen Lösemittel einen Kristallisationsinhibitor, ein viskositätserhöhendes Mittel und / oder eine pH-regulierende Substanz enthalten.

10. Verfahren zur Herstellung von mit wirkstoffhaltigen Mikroreservoiren beladenen, für transdermale therapeutische Systeme geeigneten Polymerschichten aus Polysiloxanen, **dadurch gekennzeichnet, daß** der Wirkstoff in einem ambiphilen Lösemittel, das mindestens zu 50 Gew-% aus ambiphilen organischen Lösemitteln besteht, gelöst wird, diese Lösung in einer Lösung eines Polysiloxans dispergiert wird, die resultierende Dispersion auf eine geeignete Folie beschichtet wird und das Lösemittel des Polysiloxans bei Temperaturen zwischen 25 und 100 °C, bevorzugt zwischen 30 und 80 °C entfernt wird.

## Claims

1. A transdermal therapeutic system comprising an active substance impermeable backing layer, at least one polymer layer with microreservoirs present therein, and at least one active substance dissolved therein, and a protective layer for removal before use, **characterized by** a combination of the following features:
a) the polymer fraction of the polymer layer consists to the extent of at least 70% by weight, preferably at least 80% by weight, of soluble polysiloxanes,
b) the solvent for the active substance consists to the extent of at least 50% by weight, preferably at least 80% by weight, of an ambiphilic, especially dipolar organic solvent, and
c) the ambiphilic solvent is soluble in polysiloxane to the extent of not more than about 20% by weight and is miscible with water at least up to a weight ratio of 1:3.

2. The transdermal therapeutic system as claimed in claim 1, wherein the polysiloxane is amine-resistant.

3. The transdermal therapeutic system as claimed in claim 1 or 2, wherein following production the microreservoirs are essentially free from water.

4. The transdermal therapeutic system as claimed in one or more of claims 1 to 3, wherein the polysiloxane is self-adhesive and if desired comprises at least one filler.

5. The transdermal therapeutic system as claimed in one or more of claims 1 to 4, wherein the microreservoir-containing layer is provided at least with one further self-adhesive layer, which is microreservoir-free, for anchoring on the skin and/or for anchoring with the backing layer.

6. The transdermal therapeutic system as claimed in one or more of claims 1 to 5, wherein the ambiphilic solvent is liquid at room temperature, judiciously has a boiling point under standard conditions of more than 80°C, in particular more than 110°C, is preferably diethylene glycol monoethyl ether, diethylene glycol dimethyl ether, one of the butanediols, tetrahydrofurfuryl alcohol, dipropylene glycol, propylene glycol or a mixture thereof, and is judiciously soluble to the extent of not more than 20% by weight in n-hexane or n-heptane.

7. The transdermal therapeutic system as claimed in one or more of claims 1 to 6, wherein the boiling point of the ambiphilic solvent is above that of the solvent for the polysiloxane, judiciously at least 10°C, preferably at least 30°C.

8. The transdermal therapeutic system as claimed in one or more of claims 1 - 7, wherein the maximum size of the microreservoirs does not exceed 80% of the thickness of the polymer layer, the microreservoirs having a diameter of on average 5 - 50 µm, preferably 5 - 25 µm.

9. The transdermal therapeutic system as claimed in one or more of claims 1 to 8, wherein the microreservoirs comprise, in addition to the active substance and the ambiphilic solvent, a crystallization inhibitor, a viscosity-increasing agent and/or a pH regulator.

10. A process for producing polysiloxane polymer layers which are suitable for transdermal therapeutic systems and are charged with active substance microreservoirs, which comprises dissolving the active substance in an ambiphilic solvent consisting to the extent of at least 50% by weight of ambiphilic organic solvents, dispersing this solution in a solution of a polysiloxane, coating the resulting dispersion onto an appropriate film, and removing the solvent of the polysiloxane at temperatures of between 25 and 100°C, preferably between 30 and 80°C.

## Revendications

1. Système thérapeutique transdermique comprenant une couche d'envers imperméable aux substances actives, au moins une couche de polymère avec des microréservoirs contenus dans celle-ci et au moins une substance active dissoute dans celle-ci, ainsi qu'une couche protectrice à retirer avant l'emploi, **caractérisé par** la combinaison des caractéristiques suivantes :
a) la partie polymère de la couche de polymère est constituée à raison d'au moins 70, de préférence d'au moins 80 % en poids de polysiloxanes solubles,
b) le solvant pour la substance active consiste à raison d'au moins 50, de préférence d'au moins 80 % en poids en un solvant organique ambiphile, en particulier dipolaire et
c) le solvant ambiphile n'est soluble qu'à raison de pas plus d'environ 20 % en poids dans le polysiloxane et est miscible à l'eau au moins jusqu'à un rapport pondéral de 1:3.

2. Système thérapeutique transdermique selon la revendication 1, **caractérisé en ce que** le polysiloxane est résistant aux amines.

3. Système thérapeutique transdermique selon la revendication 1 ou 2, **caractérisé en ce que** les microréservoirs sont après la préparation pratiquement exempts d'eau.

4. Système thérapeutique transdermique selon une ou plusieurs des revendications 1 à 3, **caractérisé en ce que** le polysiloxane est autoadhésif et contient éventuellement une charge.

5. Système thérapeutique transdermique selon une ou plusieurs des revendications 1 à 4, **caractérisé en ce que** la couche contenant des microréservoirs est munie d'une autre couche autoadhésive sans réservoirs pour l'accrochage sur la peau et/ou pour l'accrochage avec la couche d'envers.

6. Système thérapeutique transdermique selon une ou plusieurs des revendications 1 à 5, **caractérisé en ce que** le solvant ambiphile est liquide à la température ambiante, présente convenablement un point d'ébullition, dans les conditions normales, de plus de 80°C, en particulier de plus de 110°C, est de préférence l'éther monoéthylique de diéthylèneglycol, l'éther diméthylique de diéthylèneglycol, l'un des butanediols, l'alcool tétrahydrofurfurylique, le dipropylèneglycol, le propylèneglycol ou un mélange de ceux-ci et n'est convenablement soluble qu'à raison de pas plus de 20 % en poids dans le n-hexane ou le n-heptane.

7. Système thérapeutique transdermique selon une ou plusieurs des revendications 1 à 6, **caractérisé en ce que** le point d'ébullition du solvant ambiphile est supérieur à celui du solvant pour le polysiloxane, convenablement d'au moins 10, de préférence d'au moins 30°C.

8. Système thérapeutique transdermique selon une ou plusieurs des revendications 1 à 7, **caractérisé en ce que** la taille maximale des microréservoirs n'excède pas 80 % de l'épaisseur de la couche de polymère, les microréservoirs présentant un diamètre d'en moyenne 5-50, de préférence de 5-25 µm.

9. Système thérapeutique transdermique selon une ou plusieurs des revendications 1 à 8, **caractérisé en ce que**, outre la substance active et le solvant ambiphile, les microréservoirs contiennent un inhibiteur de cristallisation, un agent augmentant la viscosité et/ou une substance régulant le pH.

10. Procédé pour la production de couches de polymère à base de polysiloxanes, appropriées à des systèmes thérapeutiques transdermiques, chargées de microréservoirs contenant une substance active, **caractérisé en ce qu'**on dissout la substance active dans un solvant ambiphile qui consiste au moins à raison de 50 % en poids en solvants organiques ambiphiles, on disperse cette solution dans une solution d'un polysiloxane, on applique la dispersion résultante sur une pellicule appropriée et on élimine le solvant du polysiloxane à des températures comprises entre 25 et 100°C, de préférence entre 30 et 80°C.
